# FASCICULE DE BREVET EUROPEEN

(11) **EP 3 389 773 B1**
(45) Date de publication et mention de la délivrance du brevet: **29.01.2020**
(21) Numéro de dépôt: 16809092.6
(22) Date de dépôt: 14.12.2016
(51) Int. Cl.: A61N 1/372, A61N 1/375

(54) **DISPOSITIF D'AMPLIFICATION DE COMMUNICATION COMPRENANT DES ÉLÉMENTS DE MAINTIEN POUR UNE CAPSULE IMPLANTABLE**
KOMMUNIKATIONSVERSTÄRKUNGSVORRICHTUNG MIT HALTEELEMENTEN FÜR EINE IMPLANTIERBARE KAPSEL
COMMUNICATION AMPLIFICATION DEVICE COMPRISING RETENTION ELEMENTS FOR AN IMPLANTABLE CAPSULE

(30) Priorité: 17.12.2015 FR 1562572
(43) Date de publication de la demande: 24.10.2018
(73) Titulaire: Sorin CRM SAS, 92140 Clamart (FR)
(72) Inventeur: REGNIER, Willy, 91160 Longjumeau (FR); DEBROUX, Jean-François, 38590 Saint Etienne de Saint Geoirs (FR)
(74) Mandataire: Grünecker Patent- und Rechtsanwälte PartG mbB
(86) Numéro de dépôt international: PCT/EP2016/080877
(87) Numéro de publication internationale: WO 2017/102778

(56) Documents cités:
- US-A1- 2002 042 637
- US-A1- 2005 080 346
- US-A1- 2006 241 422
- US-A1- 2009 292 273
- US-A1- 2013 338 452
- US-A1- 2015 066 123

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au cœur des impulsions électriques de stimulation, de resynchronisation et/ou de défibrillation en cas de trouble du rythme détecté par le dispositif.

L'invention concerne notamment, mais de manière non limitative, ceux de ces dispositifs qui se présentent sous forme d'une capsule autonome destinée à être implantée dans une cavité cardiaque (ventricule ou oreillette, à droite ou à gauche). Ces capsules sont dépourvues de toute liaison mécanique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless*", pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

Deux catégories de capsules autonomes existent. La première catégorie concerne les capsules endocavitaires, qui sont placées dans une des cavités cardiaques. La seconde catégorie concerne les capsules épicardiques qui sont fixées sur la paroi externe du myocarde aussi appelée épicarde.

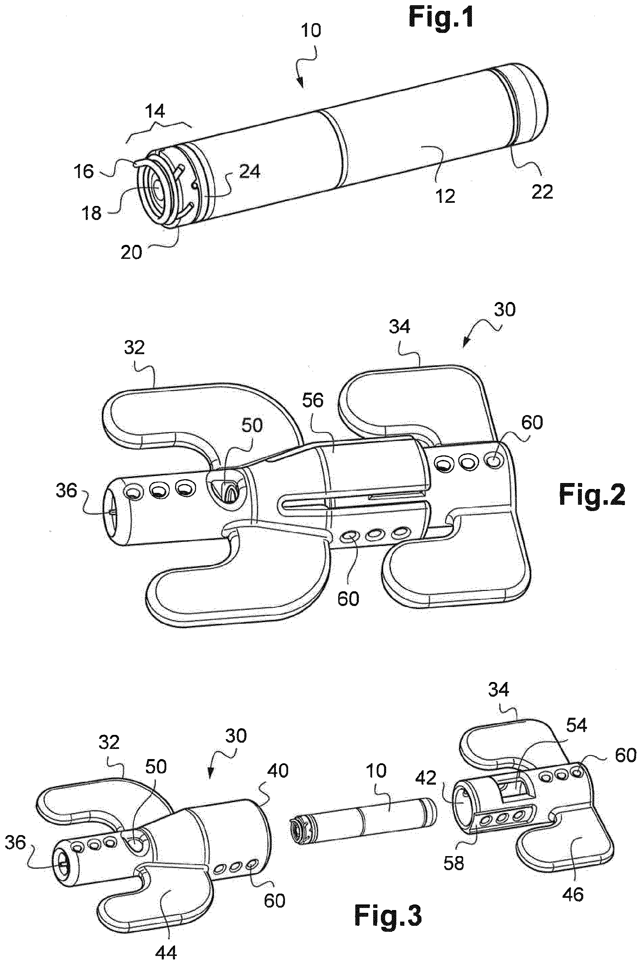

Les capsules endocavitaires ont une forme cylindrique, par exemple de gélule ou de capsule tel qu'illustré en figure 1 afin de pouvoir être introduites de manière longitudinale par un accessoire d'implantation *in situ,* tel qu'un cathéter à partir du système veineux ou artériel du patient.

A l'extrémité de la capsule se trouve un moyen de fixation afin d'ancrer la capsule au site de stimulation souhaité.

Une capsule implantable telle que décrite dans le document US 2008/088397, comprend un corps abritant les principaux éléments du dispositif (circuits électroniques, source d'énergie, électrodes de stimulation) et une embase solidaire du corps et supportant rigidement un moyen de fixation à la paroi, notamment à la paroi endocavitaire.

En outre, ces capsules comprennent un dispositif de communication permettant de communiquer avec un dispositif externe, par exemple un programmateur, par radiofréquence ou par le corps humain, notamment par communication par voie intracorporelle HBC (en terminologie anglosaxonne « Human Body Communication »), ou tout autre système, et également avec un ou plusieurs autres implants, pour la transmission et la réception d'information. Le document US 2002/042637 A1 décrit une antenne apte à être utilisée avec un microstimulateur implantable.

Concernant les capsules autonomes implantables utilisant par exemple le protocole de communication HBC, il est difficile de communiquer avec ces capsules avant leur implantation dans le patient. Une solution consiste, par exemple, lors de la fabrication d'une capsule autonome implantable, à plonger celle-ci dans un liquide conducteur et de plonger également dans ce liquide, deux électrodes reliées à un moniteur externe afin de communiquer avec la capsule. Le liquide est par exemple du sérum physiologique de type NaCI 0,9g/l.

Toutefois, cette solution permettant de communiquer avec la capsule autonome implantable présente des inconvénients. Tout d'abord, le liquide conducteur n'est pas représentatif des tissus du corps humain. Ensuite, la communication entre la capsule et le moniteur externe est dépendante de la conductivité des électrodes et des fils reliant ces électrodes au moniteur externe.

Un tel dispositif autonome implantable utilisant le protocole de communication HBC présente également l'inconvénient qu'il est impossible de vérifier le bon fonctionnement du système de la capsule et de programmer cette capsule après que la capsule ait été stérilisée et placée dans un sachet stérile.

Le bon fonctionnement du système de la capsule et sa programmation peuvent seulement être réalisés par cette méthode d'immersion par le chirurgien au moment de l'implantation de la capsule ce qui entraine des risques préopératoires et des manipulations additionnelles de la capsule autonome implantable non souhaitées lors de l'implantation de ladite capsule.

Le présent document vise à proposer un dispositif d'amplification de communication pour une capsule implantable autonome qui permette d'éviter de devoir immerger la capsule implantable afin de communiquer avec celle-ci.

Cet aspect est particulièrement critique, dans la mesure où la stérilité de la capsule implantable autonome doit être préservée, et il est primordial d'avoir une bonne communication entre la capsule implantable autonome et le dispositif externe, afin de s'assurer du bon fonctionnement de la capsule et réaliser sa programmation.

Plus précisément, le présent document propose à cet effet un dispositif d'amplification de communication pour une capsule implantable, notamment pour une capsule autonome de stimulation cardiaque, la capsule comprenant sur son extrémité distale une électrode distale apte à venir en contact avec un tissu d'une paroi d'un organe d'un patient.

Le dispositif d'amplification comprend un premier élément de maintien et un second élément de maintien de la capsule implantable, l'extrémité distale de la capsule étant apte à être insérée dans le premier élément de maintien et l'extrémité proximale étant apte à être insérée dans le second élément de maintien, ledit premier élément de maintien comprenant une antenne d'amplification de communication, ladite antenne d'amplification de communication étant apte à être couplée à l'électrode distale de la capsule. De façon caractéristique de l'invention, le premier élément de maintien et le second élément de maintien comprennent des moyens de verrouillage complémentaires aptes à maintenir bloqué le premier élément de maintien avec le second élément de maintien et des moyens de manipulation configurés pour être alignés dans un plan horizontal lorsque les éléments de maintien sont verrouillés ensemble et pour former un angle l'un par rapport à l'autre lorsque les éléments de maintien sont déverrouillés.

Un tel dispositif d'amplification de communication présente l'avantage de permettre dès la fabrication de la capsule implantable autonome de vérifier son bon fonctionnement. De plus, lors de la préparation de son implantation, la capsule implantable peut être testée et programmée sans être extraite de son emballage stérile.

Selon diverses caractéristiques subsidiaires avantageuses :
- l'antenne d'amplification de communication comprend au moins deux pièces mobiles l'une par rapport à l'autre et un élément ressort permettant la mobilité des deux pièces l'une par rapport à l'autre afin de mettre en contact l'antenne d'amplification de communication avec l'électrode distale de la capsule.
- l'antenne d'amplification de communication est formée d'une pièce conductrice souple.
- le premier élément de maintien comprend un réceptacle dans lequel est fixée l'antenne d'amplification de communication et qui comprend un espace permettant l'insertion de l'électrode distale de la capsule en vis-à-vis de l'antenne d'application de communication pour être couplée avec ladite antenne d'amplification de communication.
- la capsule comprend à son extrémité distale une vis d'ancrage comprenant des spires, et en ce que le réceptacle comprend un épaulement contre lequel viennent en appui les spires de la vis d'ancrage de la capsule.
- le premier élément de maintien comprend une ouverture permettant de visualiser le couplage réalisé entre l'antenne d'amplification de communication et l'électrode distale de la capsule.
- le premier élément de maintien et le second élément de maintien sont aptes à s'assembler l'un avec l'autre.
- le premier et/ou le second élément de maintien comprennent au moins un orifice traversant la paroi dudit élément de maintien pour permettre le passage d'un fluide dans le dispositif d'amplification.
- la capsule ayant une électrode proximale, le second élément de maintien comprend une antenne d'amplification de communication apte à être mise en contact avec l'électrode proximale de la capsule.

On va maintenant décrire un exemple de mise en œuvre de la présente invention, en référence aux dessins annexés où les mêmes références désignent d'une figure à l'autre des éléments identiques ou fonctionnellement semblables.
La Figure 1 est une vue d'ensemble en perspective d'une capsule implantable.
La Figure 2 est une vue d'ensemble en perspective d'un dispositif d'amplification de communication.
La Figure 3 est une vue détaillée des différents éléments composant un dispositif d'amplification de communication.
La Figure 4 est une vue écorchée en perspective d'un dispositif d'amplification de communication.
La Figure 5 est une vue d'une antenne de communication destinée à être insérée dans le dispositif d'amplification.
Les Figures 6a et 6b sont des vues d'un premier élément de maintien selon un mode de réalisation.
Les Figures 7a et 7b sont des vues d'un second élément de maintien selon un mode de réalisation.
La Figure 8 est une vue détaillée des différents éléments composant un dispositif d'amplification de communication selon un second mode de réalisation.
La Figure 9 est une vue d'un dispositif de test et/ou programmation d'une capsule insérée dans un dispositif d'amplification de communication.

On va maintenant décrire un exemple de réalisation de l'invention.

En référence tout d'abord à la Figure 1, il est représenté une capsule implantable 10, ici une capsule autonome de stimulation cardiaque, comprenant un corps tubulaire de capsule 12, un élément distal 14 pourvu à son extrémité distale d'un moyen d'ancrage 16, par exemple de type vis hélicoïdale et d'une électrode distale 18.

Le moyen d'ancrage 16 sous forme de vis est formé par un fil enroulé en hélice à pas de vissage à droite et est monté sur un support d'ancrage 20 intégrant des aménagements assurant l'irréversibilité de l'ancrage. Le moyen d'ancrage 16 est apte à venir en contact avec un tissu d'une paroi d'un organe d'un patient.

Selon un mode de réalisation particulier de la capsule autonome, celle-ci comprend un élément d'isolation électrique 24 inséré entre le corps 12 et l'élément distal 14 pour isoler l'élément distal dudit corps.

Le corps de la capsule 12 abrite quant à lui, un ensemble d'éléments fonctionnels de la capsule, notamment un module électronique, une batterie et comprend également une électrode proximale 22.

La Figure 2 illustre un dispositif d'amplification de communication 30 dans lequel est positionnée une capsule implantable 10 tel qu'illustrée en figure 1.

Le dispositif d'amplification de communication 30 comprend un premier élément de maintien 32 et un second élément de maintien 34 de la capsule implantable.

L'extrémité distale de la capsule 10 est apte à être insérée dans le premier élément de maintien 32 et l'extrémité proximale de la capsule est apte à être insérée dans le second élément de maintien 34 du dispositif d'amplification de communication 30.

Ledit premier élément de maintien 32 comprend une antenne d'amplification de communication 36, cette dernière étant apte à être couplée à l'électrode distale 18 de la capsule 10.

Ainsi, la capsule est d'une part maintenue dans un dispositif d'amplification de communication et d'autre part, l'antenne d'amplification de communication 36 du dispositif est en contact avec l'électrode distale 18 de la capsule afin d'amplifier le signal, notamment le signal HBC, délivré par la capsule autonome.

Ainsi, il devient possible de communiquer avec la capsule à partir d'un dispositif externe tel qu'un moniteur, avant l'implantation de la capsule, notamment lorsque l'ensemble formé par la capsule et le dispositif d'amplification est dans son emballage stérile. Il est ainsi rendu possible de vérifier le bon fonctionnement de la capsule ainsi que de procéder à sa programmation avant la réalisation de l'opération et l'implantation de la capsule dans le patient.

Le premier élément de maintien 32 et le second élément de maintien 34 de la capsule implantable sont réalisés par exemple en matière plastique.

Chacun des premier et second éléments de maintien 32, 34 présente respectivement une cavité 40, 42 telle que montrée en figure 3, ajustée à la dimension externe de la capsule de sorte à maintenir la capsule implantable dans le dispositif d'amplification 30.

Un tel ajustement, notamment de la cavité 40 du premier élément de maintien 32 avec la partie distale de la capsule présente l'avantage de maintenir le couplage entre l'électrode distale 18 de la capsule et l'antenne d'amplification de communication 36 du dispositif d'amplification.

L'antenne d'amplification de communication ainsi couplée à l'électrode distale de la capsule augmente la longueur de la pièce conductrice qu'est l'électrode distale et permet ainsi d'accroitre le rayonnement du champ électrique dans son environnement.

Le dispositif d'amplification de communication permet une communication avec la capsule autonome lorsque cet ensemble est placé dans son emballage stérile de façon simple et sécurisée, sans devoir ouvrir l'emballage stérile et manipuler la capsule.

Tel que montré en figure 2 et selon un mode de réalisation particulier, les premier et second éléments de maintien 32, 34 sont aptes à s'assembler l'un avec l'autre.

La figure 3 illustre les différents éléments du dispositif d'amplification de communication 30, à savoir le premier et le second élément de maintien 32, 34, et la capsule 10 venant s'insérer dans les éléments de maintien.

Le second élément de maintien 34 comprend une cavité 42 de forme complémentaire à la partie proximale de la capsule.

Selon le mode de réalisation illustré à la figure 1 et à la figure 3, la capsule 1 est de forme cylindrique, la cavité est donc de forme cylindrique ajustée à l'extrémité proximale de la capsule 10.

Selon un mode de réalisation particulier, afin de faciliter l'insertion de la partie proximale de la capsule dans le second élément de maintien 34, un jeu important est présent entre la partie proximale de la capsule et le contour de la cavité 42 de l'élément de maintien 34, par exemple de 2 mm.

Toutefois dans la partie basse de la cavité, le jeu entre la partie proximale de la capsule et la partie basse de la cavité est réduit de sorte que la partie proximale de la capsule soit centrée et maintenue. Pour ce faire, la partie basse de la cavité est relativement ajustée à la dimension externe de la partie proximale de la capsule. Selon un exemple, le jeu présent en partie basse de la cavité est de 0 à -0,1 mm.

Le premier élément de maintien 32 comprend également une cavité de forme complémentaire à la partie distale de la capsule, notamment de forme cylindrique ajustée à l'extrémité distale de la capsule 10. En outre, cette cavité présente une profondeur telle que la capsule est insérée jusqu'à ce que l'électrode distale 18 de la capsule vienne se coupler à l'antenne d'amplification de communication 36 positionnée dans le premier élément de maintien 32.

Selon un mode de réalisation particulier, le premier moyen de maintien comprend un jeu important entre la partie distale de la capsule et le contour de la cavité de l'élément de maintien 32, par exemple de 2 mm. Toutefois dans la partie basse de la cavité, le jeu entre la partie distale de la capsule et la partie basse de la cavité est réduit de sorte que la partie distale de la capsule soit centrée. Pour ce faire, la partie basse de la cavité est relativement ajustée à la dimension externe de la partie distale de la capsule. Selon un exemple, le jeu présent dans la partie basse de la cavité est de 0 à 0,2 mm.

La différence d'ajustement décrite ci-dessus entre le premier et le second élément de maintien et la capsule autonome permet au chirurgien de retirer le premier élément de maintien de la capsule lors de l'implantation et que cette capsule reste maintenue par le second élément de maintien permettant au chirurgien de fixer sur la capsule le dispositif de pose. Ainsi, une manipulation aisée pour le chirurgien est permise.

Comme illustré à la figure 1 et à la figure 2, le premier et/ou le second moyen de maintien 32, 34 comprennent respectivement des moyens de manipulation 44, 46 afin de permettre la prise aisée des deux éléments de maintien par le chirurgien. Les moyens de manipulation 44 et 46 sont par exemple des protubérances positionnées de part et d'autre du corps des éléments de maintien.

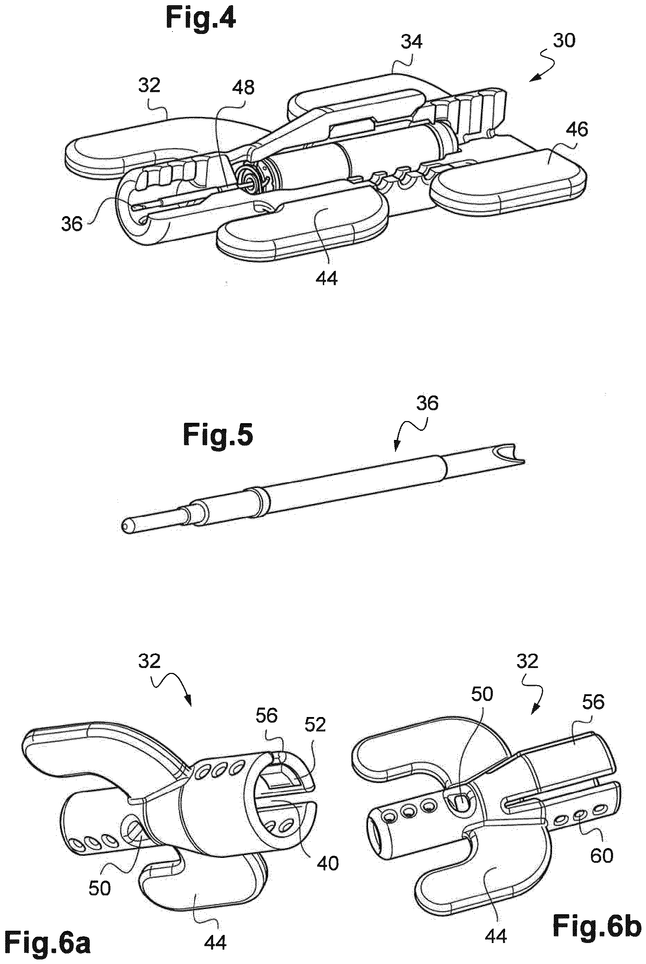

Les figures 4, 6a et 6b illustrent en détail un mode de réalisation du premier élément de maintien 32.

Le premier élément de maintien 32 comprend un réceptacle 48, au fond de la cavité 40, dans lequel est fixée l'antenne d'amplification de communication 36 et qui comprend un espace permettant l'insertion de l'électrode distale 18 de la capsule en vis-à-vis de l'antenne d'amplification de communication 36 pour être couplée avec ladite antenne d'amplification de communication 36.

La capsule telle qu'illustrée notamment en figure 1, comprend à son extrémité distale une vis d'ancrage 16 comprenant des spires.

Selon un mode de réalisation, pour faciliter la mise en contact de l'électrode distale 18 avec l'antenne d'amplification de communication 36, le premier élément de maintien 32, notamment le réceptacle 48 de ce premier élément de maintien comprend un épaulement contre lequel viennent en appui les spires de la vis d'ancrage 16 de la capsule de sorte à rapprocher les spires de la vis d'ancrage 16 entre elles et de donner un meilleur accès à l'électrode distale 18.

Le réceptacle est notamment de forme cylindrique dans lequel se loge l'épaulement. L'épaulement permet de dessiner un espace, notamment cylindrique creux dans lequel vient s'insérer l'électrode distale. L'espace créé par cet épaulement est substantiellement de même diamètre que l'électrode distale.

L'électrode distale 18 de la capsule est par exemple en forme de dôme ou de pointe ou est plan.

L'antenne d'amplification de communication 36 insérée dans le premier élément de maintien 32 comprend une extrémité insérée dans l'espace créé dans l'épaulement. L'antenne d'amplification de communication 36 est positionnée de façon à entrer en contact avec la face de l'électrode distale 18 de la capsule lorsque la capsule est selon un mode de réalisation centrée dans le premier élément de maintien 32.

La figure 5 illustre de manière détaillée un exemple de réalisation de l'antenne d'amplification de communication 36.

L'antenne d'amplification de communication, selon l'exemple illustré, comprend au moins deux pièces, par exemple cylindriques, mobiles l'une par rapport à l'autre pour permettre de mettre en contact l'antenne d'amplification de communication 36 avec l'électrode distale 18 de la capsuie lors de l'insertion de la capsule dans le premier élément de maintien 32, quel que soit la longueur de l'électrode distale 18 de la capsule.

Pour ce faire, il est prévu par exemple un élément ressort permettant la mobilité des deux pièces l'une par rapport à l'autre.

Selon un autre mode de réalisation, l'antenne d'amplification peut être réalisée d'une seule pièce conductrice souple.

L'élasticité de l'antenne, peut être réalisée au moyen d'une lame souple ou d'une matière souple, par exemple un fil conducteur souple, ou tout autre moyen compressible afin de permettre la mise en contact de l'antenne d'amplification de communication 36 avec l'électrode distale 18 de la capsule lors de l'insertion de la capsule dans le premier élément de maintien 32, quel que soit la longueur de l'électrode distale 18 de la capsule. L'antenne peut aussi par exemple être enrobée d'une matière souple telle que du silicone, l'enrobage pouvant permettre aussi l'effet ressort de l'antenne.

De préférence, l'extrémité de l'antenne d'amplification de communication 36 en contact avec l'électrode distale est sphérique afin de créer un contact ponctuel avec l'électrode distale et limiter le risque de rayure sur le revêtement de l'électrode distale, celui-ci étant en général de type nitrure de titane (TiN).

L'antenne d'amplification de communication 36 peut être montée de manière serrée dans le premier élément de maintien. Toutefois, selon d'autres modes de réalisation, l'antenne d'amplification de communication 36 peut être ajustée, collée, sertie ou soudée dans le premier élément de maintien.

Comme illustré sur les figures 2, 3, 6a et 6b, le premier élément de maintien 32 comprend au moins une ouverture 50 permettant de visualiser le couplage réalisé entre l'antenne d'amplification de communication 36 et l'électrode distale 18 de la capsule. Cette ouverture 50 permet notamment de vérifier la liaison physique entre l'antenne d'amplification de communication 36 et l'électrode distale 18 de la capsule.

Selon un mode de réalisation particulier, le premier et le second élément de maintien 32, 34 sont conçus de sorte à s'assembler l'un avec l'autre, notamment par insertion d'une partie d'un élément de maintien dans l'autre élément de moyen. Pour ce faire, les géométries, notamment du corps du second élément de maintien et du corps du premier élément de maintien, doivent être complémentaires avec, par exemple un jeu fonctionnel de 0,1 mm.

La figure 2 illustre les deux éléments de maintien assemblés. Conformément à l'invention, afin de maintenir fermement les deux éléments de maintien assemblés, les premier et second éléments de maintien 32, 34 comprennent des moyens de verrouillage complémentaires aptes à maintenir bloqué le premier élément de maintien avec le second élément de maintien.

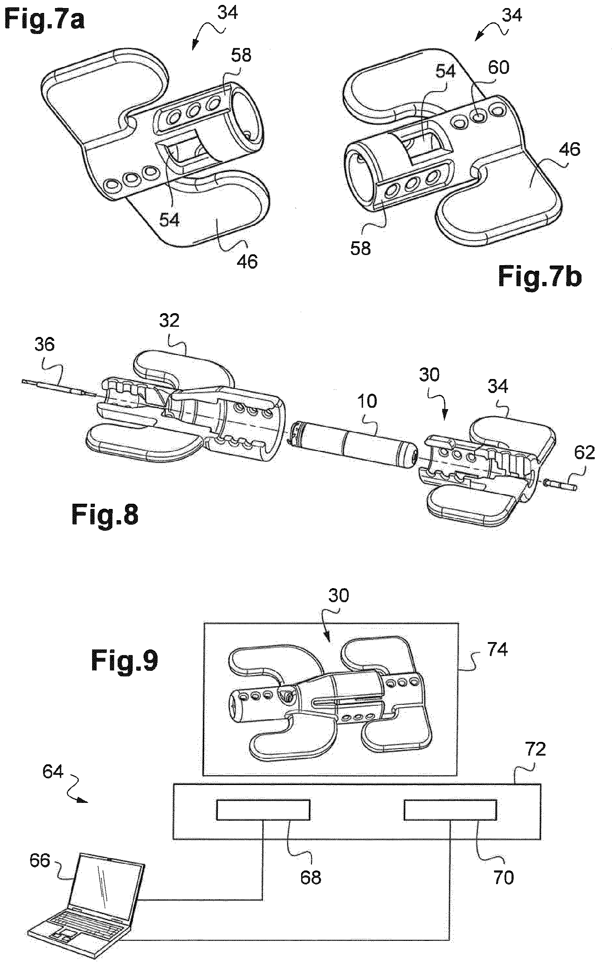

Selon un exemple de réalisation, les moyens de verrouillage 52 du premier élément de maintien sont illustrés en figures 6a et 6b et les moyens de verrouillage 54 du second élément de maintien sont illustrés en figures 7a et 7b.

Les moyens de verrouillage 52 du premier élément de maintien 32 sont par exemple un clip positionné sur la paroi interne de la cavité 40 présente dans le corps de l'élément de maintien accueillant la partie distale de la capsule et les moyens de verrouillage 54 complémentaires du second élément de maintien 54 sont par exemple une ouverture, de préférence traversant, de forme complémentaire au clip, réalisée dans la paroi de la cavité du corps du second élément de maintien 54 accueillant la partie proximale de la capsule.

Selon une forme particulière de réalisation, le premier élément de maintien comprend deux ouvertures de part et d'autre des moyens de verrouillage, notamment du clip, de sorte à créer une lame 56 élastiquement déformable s'étendant du haut de la cavité 40 vers la partie basse de la cavité et d'une largeur légèrement supérieure à la largeur du clip.

Tel qu'illustré aux figures 7a et 7b, le second élément de maintien comprend au moins une rainure 58 permettant au clip 52 du premier moyen de maintien de coulisser sur une longueur donnée, la longueur de la rainure, lors de l'assemblage des deux éléments de maintien.

Ladite au moins une rainure 58 du second élément de maintien est positionnée par exemple à 90° par rapport au moyen de verrouillage 54 du second élément de maintien.

La rainure 58 présente une certaine longueur de sorte à régler la profondeur d'assemblage du second élément de maintien 34 dans le premier élément de maintien 32.

L'assemblage des premier et second éléments de maintien est réalisé selon le procédé maintenant décrit.

Le second élément de maintien est assemblé avec le premier élément de maintien en faisant coulisser la ou les lames 56 du premier élément de maintien 32 dans la ou les rainures 58 du second élément de maintien, par coopération du clip avec la rainure, jusqu'à atteindre l'extrémité de la rainure 58. Ensuite, le premier élément de maintien est pivoté par rapport au second élément de maintien jusqu'à faire coopérer les moyens de verrouillage respectif des éléments de maintien, par exemple afin de faire coopérer le clip 52 du premier élément de maintien avec l'ouverture 54 correspondante dans le second élément de maintien.

Le désassemblage des deux éléments de maintien s'effectue en sens inverse, c'est-à-dire par rotation du premier élément de maintien par rapport au second élément de maintien jusqu'à ce que le clip 52 de verrouillage coopère avec la rainure 58 du second élément de maintien, puis par une translation axiale du premier élément de maintien par rapport au second élément de maintien.

Selon une variante de réalisation, le désassemblage des deux éléments de maintien s'effectue par une translation axiale unique du premier élément de maintien par rapport au second élément de maintien.

En outre, conformément à l'invention, afin de guider le chirurgien dans sa manipulation du dispositif d'amplification de communication, les moyens de manipulation sont alignés dans un plan horizontal lorsque les premier et second éléments de maintien sont verrouillés ensemble et les moyens de manipulation 44, 46 forment un angle l'un par rapport à l'autre lorsque les premier et second éléments de maintien sont déverrouillés.

Selon un mode de réalisation, l'ouverture 54 réalisée dans le second moyen de maintien, apte à accueillir le clip 52 du premier moyen de maintien, comprend des chanfreins sauf sur sa face avant afin d'augmenter la force nécessaire au désengagement du clip 52 de verrouillage.

Tel qu'illustré sur la figure 2, au moins l'un des éléments de maintien, de préférence les deux éléments de maintien, comprennent au moins un orifice 60 traversant la paroi de la cavité d'accueil de la capsule du ou des éléments de maintien pour permettre le passage d'un fluide dans le dispositif d'amplification de communication, notamment dans les premiers et second moyens de maintien.

En effet, afin de décontaminer la capsule et les éléments de maintien, l'ensemble une fois assemblé, est plongé dans un fluide de décontamination. Les orifices 60 présents dans la paroi des éléments de maintien permettent un passage du fluide sur l'ensemble de la capsule.

Selon un mode de réalisation particulier dans lequel des orifices 60 traversant sont réalisés au fond de la rainure 58 du second élément de maintien, alors des orifices 60 traversant correspondant sont réalisés sur la paroi du premier élément de maintien, de sorte que, une fois assemblé, les orifices 60 réalisés au fond de la rainure correspondent sensiblement aux orifices 60 réalisés sur la paroi du premier élément de maintien.

Selon un mode de réalisation particulier de l'invention, le second élément de maintien apte à accueillir la partie proximale de la capsule, comprend aussi une seconde antenne d'amplification de communication 62 apte à être mise en contact avec l'élément arrière de la capsule qui supporte l'électrode proximale de la capsule, tel qu'illustré en figure 8.

Pour ce faire, et selon un mode de réalisation particulier, la seconde antenne d'amplification de communication 62 est positionnée dans le second élément de maintien et maintenue dans l'axe de positionnement de la capsule 10.

Un tel dispositif d'amplification de communication 30 permettant d'amplifier le signal de la première électrode 18 et de la seconde électrode 22 de la capsule permet de tester efficacement la capsule insérée dans les premier et second moyens de maintien.

Selon un exemple de réalisation, la seconde antenne d'amplification de communication 62 est plus petite que la première antenne d'amplification de communication 36 apte à être connectée à l'électrode distale, notamment du fait que l'électrode proximale 22 de la capsule a une surface plus grande, environ 40 mm² et une largeur plus grande aussi, environ 2 mm² pour l'électrode distale 18.

La figure 9 illustre un dispositif de test et/ou de programmation 64 de la capsule insérée dans un dispositif d'amplification de communication 30.

Ce dispositif de test et/ou de programmation 64 comprend un moniteur externe 66 auquel deux électrodes de communication 68 et 70 sont reliées. Les électrodes de communication 68 et 70 sont espacées l'une de l'autre d'environ 20 mm et insérées dans un support d'électrodes 72 afin de positionner efficacement la capsule insérée dans le dispositif d'amplification au-dessus des électrodes de communication.

Selon un exemple de réalisation du dispositif de test et/ou de programmation 64, les électrodes de communication 68 et 70 ont une surface sensiblement carrée de 25 mm de côté.

Si l'on considère, selon un exemple donné, que le dispositif de test et/ou de programmation 64 comprend un récepteur sensible à un niveau de signal émis de -100dB/signal émis alors un couplage de -60 à -80dB est suffisant pour une bonne communication entre le dispositif de test et/ou de programmation 64 et la capsule 10 insérée dans le dispositif d'amplification de communication 30.

Des tests ont montré qu'une longueur d'antenne de 10 à 20 mm est suffisante pour assurer la distance de communication de 30 mm entre la capsule insérée dans le dispositif d'amplification de communication et les électrodes de communication.

La figure 9 illustre un tel dispositif de test et/ou de programmation 64 sur lequel un dispositif d'amplification de communication intégrant une capsule, l'ensemble étant positionné dans un sachet stérile 74, est positionné à une distance d'environ 3 mm en vis-à-vis des électrodes de communication.

## Revendications

1. Dispositif d'amplification de communication (30) pour une capsule implantable (10), notamment pour une capsule autonome de stimulation cardiaque, la capsule comprenant sur son extrémité distale une électrode distale apte à venir en contact avec un tissu d'une paroi d'un organe d'un patient, le dispositif d'amplification (30) comprenant un premier élément de maintien (32) et un second élément de maintien de la capsule implantable (34), le premier élément de maintien étant apte à recevoir une extrémité distale de la capsule apte à être insérée dans le premier élément de maintien et le second élément de maintien étant apte à recevoir une extrémité proximale de la capsule apte à être insérée dans le second élément de maintien, ledit premier élément de maintien comprenant une antenne d'amplification de communication (36), ladite antenne d'amplification de communication étant apte à être couplée à l'électrode distale de la capsule ; **caractérisé en ce que** le premier élément de maintien et le second élément de maintien comprennent des moyens de verrouillage complémentaires aptes à maintenir bloqué le premier élément de maintien avec le second élément de maintien et **en ce que** le premier élément de maintien et le second de maintien comprennent des moyens de manipulation, les moyens de manipulation des premier et second éléments de maintien étant configurés pour être alignés entre eux dans un plan horizontal lorsque les premier et second éléments de maintien sont verrouillés ensemble, et les moyens de manipulation des premier et second éléments de maintien étant configurés pour former un angle l'un par rapport à l'autre lorsque les premier et second éléments de maintien sont déverrouillés.

2. Dispositif d'amplification de communication selon la revendication 1, **caractérisée en ce que** l'antenne d'amplification de communication comprend au moins deux pièces mobiles l'une par rapport à l'autre et un élément ressort permettant la mobilité des deux pièces l'une par rapport à l'autre afin de mettre en contact l'antenne d'amplification de communication avec l'électrode distale de la capsule.

3. Dispositif d'amplification de communication selon la revendication 1, **caractérisée en ce que** l'antenne d'amplification de communication est formée d'une pièce conductrice souple.

4. Dispositif d'amplification de communication selon l'une quelconque des revendications précédentes, **caractérisé en ce que** le premier élément de maintien comprend un réceptacle dans lequel est fixée l'antenne d'amplification de communication et qui comprend un espace permettant l'insertion d'une électrode distale d'une capsule en vis-à-vis de l'antenne d'amplification de communication de sorte que ladite antenne d'amplification de communication est configurée pour être couplée à l'électrode distale de la capsule.

5. Dispositif d'amplification de communication selon la revendication 4, **caractérisé en ce que** le réceptacle comprend un épaulement configuré pour que des spires d'une vis d'ancrage à une extrémité distale d'une capsule viennent en appui contre ledit épaulement du réceptacle.

6. Dispositif d'amplification de communication selon l'une quelconque des revendications précédentes, **caractérisé en ce que** le premier élément de maintien comprend une ouverture permettant de visualiser le couplage réalisé entre l'antenne d'amplification de communication et l'électrode distale de la capsule.

7. Dispositif d'amplification de communication selon l'une quelconque des revendications précédentes, **caractérisé en ce que** le premier élément de maintien et le second élément de maintien ont des géométries complémentaires permettant l'assemblage du premier élément de maintien et du second élément de maintien l'un avec l'autre.

8. Dispositif d'amplification de communication selon l'une quelconque des revendications précédentes, **caractérisé en ce que** le premier et/ou le second élément de maintien comprennent au moins un orifice traversant une paroi dudit élément de maintien pour permettre le passage d'un fluide dans le dispositif d'amplification.

9. Dispositif d'amplification de communication selon l'une quelconque des revendications précédentes, **caractérisé en ce que** le second élément de maintien comprend une antenne d'amplification de communication (32) apte à être mise en contact avec une électrode proximale d'une capsule.

10. Assemblage d'une capsule implantable et d'un dispositif d'amplification de communication selon l'une des revendications 1 à 9, dont la capsule (30) comprend sur son extrémité distale une électrode distale (18) apte à venir en contact avec un tissu d'une paroi d'un organe d'un patient.

## Patentansprüche

1. Kommunikationsverstärkungsvorrichtung (30) für eine implantierbare Kapsel (10), insbesondere für eine autonome Herzstimulationskapsel, wobei die Kapsel an ihrem distalen Ende eine distale Elektrode umfasst, die mit einem Gewebe einer Wand eines Organs eines Patienten in Kontakt kommen kann,
die Verstärkungsvorrichtung (30) ein erstes Halteelement (32) und ein zweites Halteelement der implantierbaren Kapsel (34) umfasst, wobei das erste Halteelement angepasst ist, um ein distales Ende der Kapsel aufzunehmen, das angepasst ist, um in das erste Halteelement eingeführt zu werden, und das zweite Halteelement angepasst ist, um ein proximales Ende der Kapsel zu empfangen, das angepasst ist, um in das zweite Halteelement eingeführt zu werden, wobei das erste Halteelement eine Kommunikationsverstärkungsantenne (36) umfasst, wobei die Kommunikationsverstärkungsantenne angepasst ist, um mit der distalen Elektrode der Kapsel gekoppelt zu werden;
**dadurch gekennzeichnet, dass** das erste Halteelement und das zweite Halteelement zusätzliche Verriegelungsmittel umfassen, die in der Lage sind, das erste Halteelement mit dem zweiten Halteelement zu verriegeln und
dass das erste Halteelement und das zweite Halteelement Handhabungsmittel umfassen, wobei die Handhabungsmittel des ersten und zweiten Halteelements konfiguriert sind, um in einer horizontalen Ebene miteinander ausgerichtet zu werden, wenn das erste und das zweite Halteelement miteinander verriegelt sind, und die Handhabungsmittel des ersten und zweiten Halteelements konfiguriert sind, um einen Winkel zueinander zu bilden, wenn das erste und zweite Halteelement entriegelt sind.

2. Kommunikationsverstärkervorrichtung nach Anspruch 1, **dadurch gekennzeichnet, dass** die Kommunikationsverstärkungsantenne mindestens zwei relativ zueinander bewegliche Teile und ein Federelement umfasst, das die Mobilität der beiden Teile relativ zueinander ermöglicht, um die Kommunikationsverstärkungsantenne mit der distalen Elektrode der Kapsel zu kontaktieren.

3. Kommunikationsverstärkervorrichtung nach Anspruch 1, **dadurch gekennzeichnet, dass** die Kommunikationsverstärkungsantenne aus einem flexiblen leitenden Teil gebildet ist.

4. Kommunikationsverstärkungsvorrichtung nach einem der obigen Ansprüche, **dadurch gekennzeichnet, dass** das erste Halteelement eine Aufnahme umfasst, in der die Kommunikationsverstärkungsantenne befestigt ist und die einen Raum umfasst, der das Einsetzen einer distalen Elektrode einer Kapsel gegenüber der Kommunikationsverstärkungsantenne ermöglicht, so dass die Kommunikationsverstärkungsantenne konfiguriert ist, um mit der distalen Elektrode der Kapsel gekoppelt zu werden.

5. Kommunikationsverstärkungsvorrichtung nach Anspruch 4, **dadurch gekennzeichnet, dass** die Aufnahme eine Schulter umfasst, die so konfiguriert ist, dass Windungen einer Ankerschraube an einem distalen Ende einer Kapsel gegen die Schulter der Aufnahme abstützen.

6. Kommunikationsverstärkungsvorrichtung nach einem der obigen Ansprüche, **dadurch gekennzeichnet, dass** das erste Halteelement eine Öffnung zum Visualisieren der Kopplung zwischen der Kommunikationsverstärkungsantenne und der distalen Elektrode der Kapsel umfasst.

7. Kommunikationsverstärkungsvorrichtung nach einem der vorhergehenden Ansprüche, **dadurch gekennzeichnet, dass** das erste Halteelement und das zweite Halteelement komplementäre Geometrien aufweisen, die die Anordnung des ersten Halteelements und des zweiten Halteelements miteinander ermöglichen.

8. Kommunikationsverstärkungsvorrichtung nach einem der obigen Ansprüche, **dadurch gekennzeichnet, dass** das erste und/oder zweite Halteelement mindestens eine Öffnung umfasst, die durch eine Wand des Halteelements verläuft, um es einem Fluid zu ermöglichen, durch die Verstärkungsvorrichtung zu gelangen.

9. Kommunikationsverstärkungsvorrichtung nach einem der obigen Ansprüche, **dadurch gekennzeichnet, dass** das zweite Halteelement eine Kommunikationsverstärkungsantenne (32) umfasst, die mit einer proximalen Elektrode einer Kapsel in Kontakt gebracht werden kann.

10. Anordnung einer implantierbaren Kapsel und einer Kommunikationsverstärkungsvorrichtung nach einem der Ansprüche 1 bis 9, deren Kapsel (30) an ihrem distalen Ende eine distale Elektrode (18) umfasst, die in der Lage ist, von einer Wand eines Organs eines Patienten mit Gewebe in Kontakt zu kommen.

## Claims

1. Communication amplification device (30) for an implantable capsule (10), in particular for an autonomous cardiac stimulation capsule, the capsule comprising on its distal end a distal electrode capable of coming into contact with a tissue of a wall of an organ of a patient,
the amplification device (30) comprising a first retention member (32) and a second retention member of the implantable capsule (34), the first retention member being adapted to receive a distal end of the capsule adapted to be inserted into the first retention member and the second retention member being adapted to receive a proximal end of the capsule adapted to be inserted into the second retention member, said first retention member comprising a communication amplification antenna (36), said communication amplification antenna being adapted to be coupled to the distal electrode of the capsule;
**characterised in that** the first retention element and the second retention element comprise additional locking means capable of locking the first retention element with the second retention element and
**in that** the first retention element and the second retention element comprise handling means, the handling means of the first and second retention elements being configured to be aligned with each other in a horizontal plane when the first and second retention elements are locked together, and the handling means of the first and second retention elements being configured to form an angle with respect to each other when the first and second retention elements are unlocked.

2. A communication amplifier device according to claim 1, **characterised in that** the communication amplifier antenna comprises at least two parts movable relative to each other and a spring element allowing the mobility of the two parts relative to each other in order to contact the communication amplifier antenna with the distal electrode of the capsule.

3. A communication amplifier device according to claim 1, **characterised in that** the communication amplifier antenna is formed of a flexible conductive part.

4. A communication amplification device according to any of the above claims, **characterised in that** the first retention element comprises a receptacle in which the communication amplification antenna is fixed and which comprises a space allowing the insertion of a distal electrode of a capsule opposite the communication amplification antenna so that said communication amplification antenna is configured to be coupled to the distal electrode of the capsule.

5. A communication amplification device according to claim 4, **characterised in that** the receptacle comprises a shoulder configured so that turns of an anchor screw at a distal end of a capsule support against said shoulder of the receptacle.

6. A communication amplification device according to any of the above claims, **characterised in that** the first retention element comprises an opening for visualizing the coupling between the communication amplification antenna and the distal electrode of the capsule.

7. A communication amplification device according to any of the preceding claims, **characterised in that** the first retention element and the second retention element have complementary geometries allowing the assembly of the first retention element and the second retention element with each other.

8. A communication amplification device according to any of the above claims, **characterised in that** the first and/or second retention element comprises at least one orifice passing through a wall of said retention element to allow a fluid to pass through the amplification device.

9. A communication amplification device according to any of the above claims, **characterised in that** the second retention element comprises a communication amplification antenna (32) capable of being brought into contact with a proximal electrode of a capsule.

10. An assembly of an implantable capsule and a communication amplification device according to one of claims 1 to 9, the capsule (30) of which comprises on its distal end a distal electrode (18) capable of coming into contact with tissue from a wall of an organ of a patient.
